# DEMANDE DE BREVET EUROPEEN

(11) **EP 0 982 014 A1**
(43) Date de publication de la demande: **01.03.2000**
(21) Numéro de dépôt: 99402134.3
(22) Date de dépôt: 26.08.1999
(51) Int. Cl.: A61F 7/10

(54) **Article réfrigérant pour la jambe et procédé d'utilisation d'un tel article**

(30) Priorité: 27.08.1998 FR 9810783
(71) Demandeur: Langevin, Yann, 75006 Paris (FR)
(72) Inventeur: Langevin, Yann, 75006 Paris (FR)
(74) Mandataire: Corret, Hélène

(57) **Abrégé**

L'invention concerne un article réfrigérant pour la jambe ainsi qu'un procédé d'utilisation d'un tel article.

Selon l'invention, cet article comporte au moins deux compartiments (1, 2, 3) indépendants, de forme allongée, réalisés en une matière souple et reliés entre eux par leur plus grande dimension, de façon à pouvoir conformer ledit article à la forme de la jambe (J), l'article comprenant en outre des moyens de fixation (60, 70) pour le maintenir en position sur la jambe (J), caractérisé en ce que les compartiments sont étanches, chacun d'eux définissant une enceinte susceptible de recevoir un fluide et comportant un moyen de fermeture (11, 21, 31) amovible de ladite enceinte.

L'invention s'applique à la relaxation des jambes de patients souffrants d'insuffisance veineuse.

## Description

La présente invention concerne un article réfrigérant pour la jambe, destiné à relaxer les membres inférieurs et à soulager la gène occasionnée notamment par une insuffisance veineuse.

On connaît déjà des produits destinés à réchauffer ou refroidir une partie du corps.

Cependant, aucun d'entre eux n'est spécifiquement adapté à la forme de la jambe.

De plus, tous les produits connus de ce type sont préalablement remplis de liquide réfrigérant. Ils sont donc relativement lourds et encombrants.

Ceci constitue un inconvénient pour les utilisateurs qui souhaitent avoir ces produits à disposition, lors de leurs déplacements.

Par ailleurs, les coûts de transport et de stockage de ces produits sont relativement importants.

L'invention a pour but de pallier ces inconvénients en proposant un article réfrigérant pour la jambe dont la forme est bien adaptée à la jambe et qui peut être facilement transporté.

Ainsi, l'invention concerne un article réfrigérant pour la jambe comprenant au moins deux compartiments indépendants de forme allongée, réalisés en une matière souple et reliés entre eux par leur plus grande dimension, de façon à pouvoir conformer ledit article à la forme de la jambe, ledit article comprenant en outre des moyens de fixation pour le maintenir en position sur la jambe, caractérisé en ce que lesdits compartiments sont étanches, chacun d'eux définissant une enceinte susceptible de recevoir directement un fluide et comportant un moyen de fermeture amovible de ladite enceinte.

De préférence, des cloisons sont prévues dans au moins un compartiment à proximité d'un bord transversal destiné à être situé à la partie inférieure de la jambe, lorsque l'article réfrigérant est en place.

Dans un premier mode de réalisation, lesdits compartiments sont fixés sur un support souple.

Dans un autre mode de réalisation lesdits compartiments sont articulés entre eux selon leur plus grande dimension.

De façon préférée, la face interne des compartiments, destinée à être en contact avec la jambe est recouverte d'une protection anti-brûlures.

De façon préférée, les moyens de fixation sont placés aux extrémités libres du support souple ou de la protection anti-brûlures.

Ces moyens de fixation peuvent notamment être constitués par deux bandes longitudinales du type velours crochet présentant une forme légèrement concave.

De manière avantageuse, les compartiments présentent une forme légèrement courbée pour mieux adapter l'article réfrigérant à la forme de la jambe.

L'invention concerne également un procédé d'utilisation de cet article réfrigérant pour la jambe, consistant, avant mise en place sur la jambe, à :
- remplir les compartiments dudit article avec un fluide et fermer lesdits compartiments et
- placer ledit article dans un réfrigérateur ou congélateur pour refroidir ledit fluide.

De préférence, le fluide utilisé pour remplir lesdits compartiments est un liquide tel que de l'eau, un liquide de refroidissement du type glycol ou encore un mélange d'eau et d'alcool.

L'invention sera mieux comprise et d'autres buts, avantages et caractéristiques de celle-ci apparaîtront plus clairement à la lecture de la description qui suit et qui est faite en relation avec les dessins annexés sur lesquels :
- la figure 1 est une vue générale en plan de l'article réfrigérant selon l'invention ;
- la figure 2 comporte les figures 2a et 2b qui sont des vues générales de l'article selon l'invention, lors de sa mise en place sur la jambe d'un patient (figure 2a) et une fois en place sur ladite jambe (figure 2b) ;
- la figure 3 est une vue en coupe transversale d'un premier mode de réalisation de l'article selon l'invention, au cours de sa mise en place sur la jambe ;
- la figure 4 est une vue similaire à la figure 3, dans laquelle l'article réfrigérant selon l'invention est en place sur la jambe ;
- la figure 5 est une vue en coupe transversale d'un deuxième mode de réalisation de l'article réfrigérant selon l'invention, au cours de sa mise en place sur la jambe d'un patient ; et
- la figure 6 est une vue similaire à la figure 5, dans laquelle l'article réfrigérant selon l'invention est mis en place sur la jambe.

Les éléments communs aux différentes figures seront désignés par les mêmes repères.

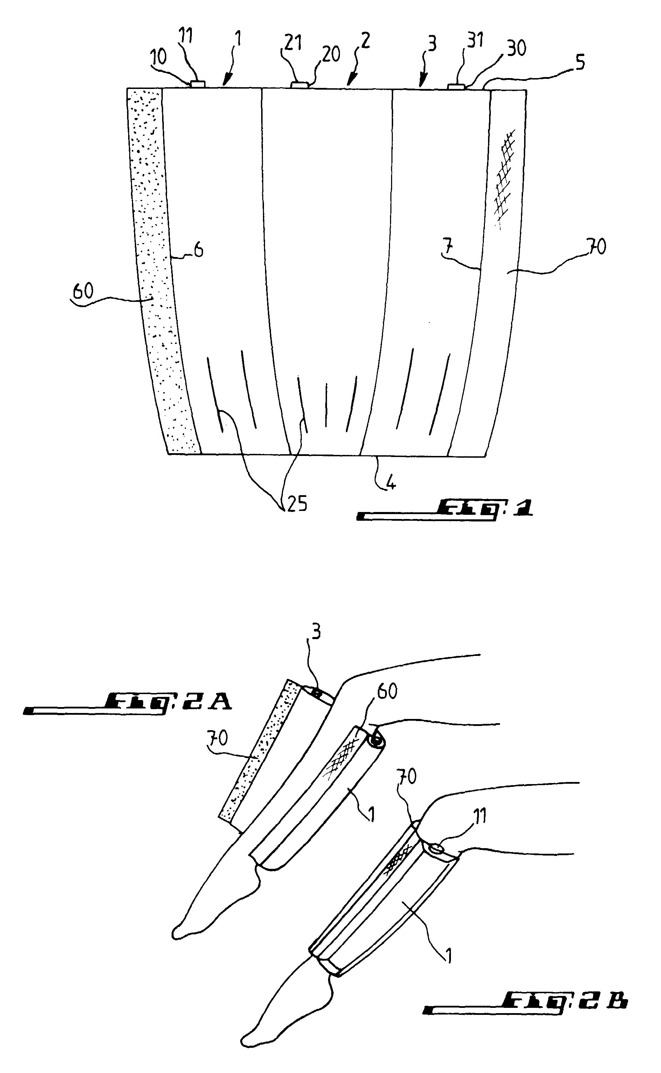

On se réfère tout d'abord à la figure 1 qui représente un exemple d'un article réfrigérant selon l'invention.

Cet article est composé de trois compartiments indépendants qui présentent une forme allongée.

Ces compartiments sont réalisés en une matière souple et ils sont étanches.

Dans l'exemple de réalisation illustré à la figure 1, les compartiments 1 à 3 présentent une largeur sur le bord transversal 4 qui est inférieure à leur largeur sur le bord transversal 5 et donc une forme effilée.

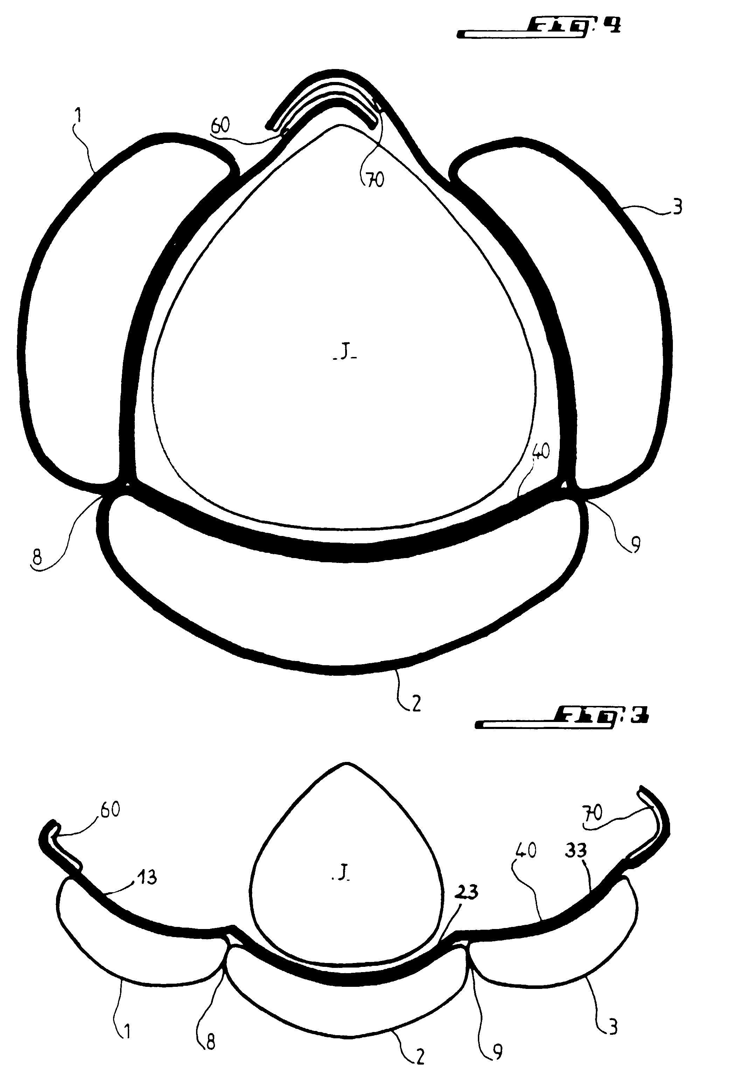

Ainsi, l'article réfrigérant selon l'invention présente une forme sensiblement tronconique en position enroulée comme illustrée à la figure 2b. Cette forme tronconique en position enroulée est également obtenue lorsque seul le compartiment central présente une forme effilée. Comme on le verra à la suite de la description, cette forme particulière permet de mieux adapter l'article à la jambe du patient.

A proximité du bord transversal 4, destiné à être placé à la partie inférieure de la jambe, sous le mollet du patient, lorsque l'article réfrigérant est utilisé, les parois des compartiments sont fixées ensemble selon des lignes sensiblement verticales pour former des cloisons 25. La fixation des parois entre elles est notamment obtenue par thermosoudure. La fonction de ces cloisons 25 qui s'étendent sur une partie de la hauteur de l'article réfrigérant, par exemple environ le quart de cette hauteur, sera expliquée dans la suite de la description.

Chaque compartiment comporte un orifice 10, 20 ou 30 qui est fermé par un moyen de fermeture amovible, tel qu'un bouchon 11, 21, 31.

Ainsi, chaque compartiment définit une enceinte étanche susceptible de recevoir directement un fluide et qui peut être individuellement remplie et obturée par un bouchon amovible.

Par ailleurs, les compartiments sont indépendants les uns des autres et étanches entre eux, puisqu'il n'existe pas de communication entre les différents compartiments.

Enfin, l'article réfrigérant représenté à la figure 1 comporte sur ses côtés longitudinaux 6 et 7, des moyens permettant de le fixer sur la jambe d'un patient.

Dans cet exemple, les moyens de fixation sont constitués par des bandes 60, 70 du type velours crochet, chacune d'elle étant fixée sur un bord longitudinal 6, 7 dudit article.

Comme l'illustre la figure 1, les bandes 60, 70 présentent de préférence une forme légèrement concave, la concavité étant tournée vers l'extérieur de l'article réfrigérant, pour que l'article réfrigérant puisse facilement s'adapter à différentes morphologies.

L'article réfrigérant selon l'invention est utilisé de la façon suivante.

Tout d'abord, chacun des compartiments 1, 2, 3 de l'article est directement rempli par un fluide, c'est-à-dire que le fluide n'est pas au préalable conditionné dans une poche par exemple avant d'être placé dans un compartiment, puis chaque compartiment est fermé grâce à un bouchon 11, 21, 31.

Ce fluide peut être notamment constitué par de l'eau, un mélange d'eau et d'alcool ou encore par un liquide de refroidissement du type glycol.

Selon le type de fluide utilisé, l'article réfrigérant selon l'invention est placé dans un réfrigérateur ou un congélateur pour refroidir le fluide présent dans les compartiments 1 à 3.

Ensuite, comme l'illustrent les figure 2a et 2b, l'article réfrigérant est placé autour de la jambe d'un patient, de façon à ce que la plus grande dimension des compartiments ou encore la dimension longitudinale s'étende le long de la jambe du patient.

Les compartiments étant réalisés en une matière souple, ils peuvent facilement s'adapter au contour de la jambe. Cette adaptation est facilitée par la forme tronconique de l'article réfrigérant en position enroulée.

L'article réfrigérant selon l'invention est ensuite maintenu en position sur la jambe, grâce aux moyens de fixation, ici constitués par les bandes longitudinales du type velours crochet 60, 70 que l'on superpose de manière appropriée.

Ainsi, lorsque l'article réfrigérant selon l'invention est en place sur la jambe d'un patient, il permet de la rafraîchir sans empêcher le patient de se déplacer.

Par ailleurs, la présence des cloisons 25 permet d'éviter que le fluide présent dans les compartiments ne s'accumule dans la partie basse de l'article, c'est-à-dire au-dessous du mollet du patient lorsque l'article est en place sur lui. Les cloisons 25 assurent donc une meilleure répartition du fluide dans les compartiments de l'article réfrigérant.

Lorsqu'on le souhaite, et après avoir oté l'article réfrigérant de la jambe du patient, les bouchons 11, 21, 31 peuvent être retirés pour vider chaque compartiment du fluide qu'il contient.

On se réfère maintenant aux autres figures qui illustrent deux modes de réalisation particuliers de l'invention. Dans les deux cas, l'article réfrigérant selon l'invention comporte trois compartiments, comme celui illustré aux figures 1 et 2. Cependant, l'invention n'est pas limitée à cette configuration. Il suffit que l'article réfrigérant selon l'invention comporte au moins deux compartiments indépendants pour faciliter son adaptation à la forme de la jambe.

Par ailleurs, la présence de plusieurs compartiments permet d'améliorer la répartition du liquide réfrigérant dans l'article selon l'invention.

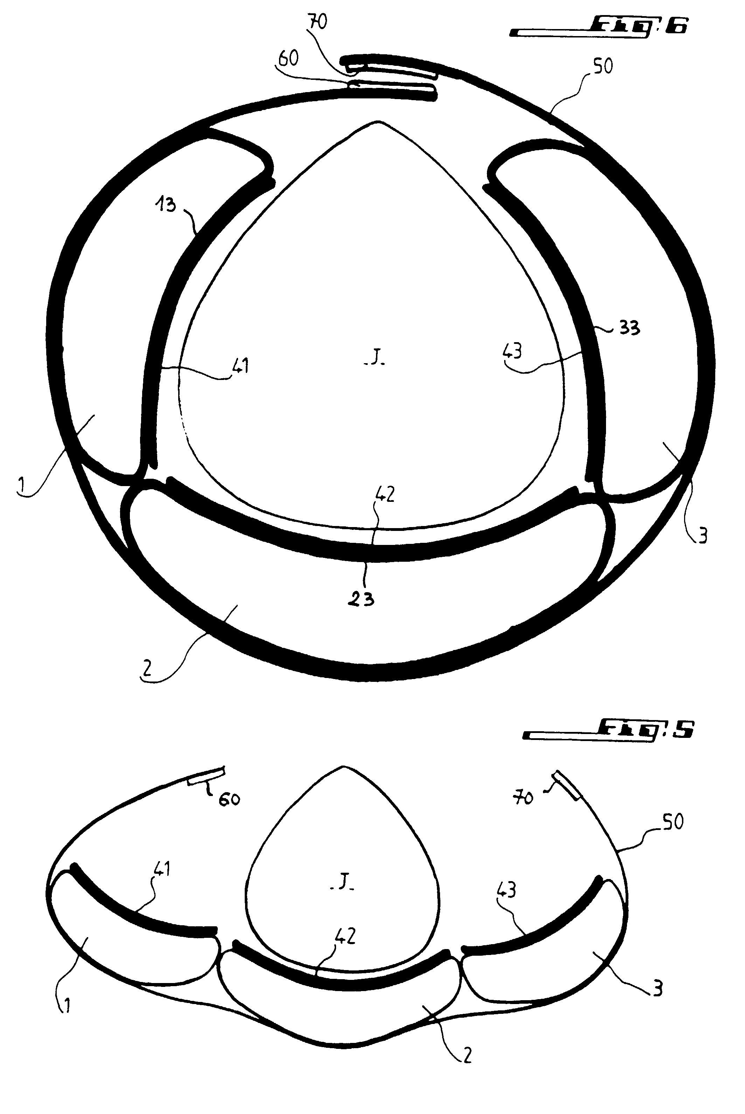

Dans le premier mode de réalisation illustré aux figures 3 et 4, l'article réfrigérant comporte un compartiment central 2 et deux compartiments adjacents 1 et 3. Les compartiments 1 et 3 présentent une forme sensiblement identique, tandis que le compartiment 2 présente une dimension transversale plus importante de façon à s'adapter à l'arrière de la jambe.

Les trois compartiments sont articulés entre eux selon leur plus grande dimension qui correspond à une dimension longitudinale.

Ces lignes d'articulation sont référencées par les repères 8 et 9.

Chacun des compartiments 1, 2 et 3 comporte de préférence une forme légèrement courbée, pour mieux s'adapter au contour de la jambe.

Comme le montre la figure 3, lors de la mise en place de l'article sur une jambe J d'un patient, le compartiment central 2 est placé sur l'arrière de la jambe J, sa face interne concave 23 épousant la forme de la jambe.

L'article réfrigérant est ensuite replié sur la jambe, de telle sorte que les compartiments 1 et 2 sont placés sur les côtés de la jambe J, leurs faces concaves 13 et 33 étant contre la jambe.

L'article est ensuite maintenu en place grâce aux moyens de fixation 60 et 70 qui sont des bandes du type velours crochet.

D'autres moyens de fixation pourraient bien sûr être prévus, par exemple des pressions ou encore des rubans susceptibles d'être noués.

De préférence, sur la face intérieure de l'article réfrigérant, formé par les faces concaves 13, 23, 33 des compartiments 1 à 3, sont disposés des moyens 40 pour protéger la jambe J de brûlures éventuelles provoquées par le froid.

Ces moyens 40 peuvent être constitués par un voile de tissu ou un film en une matière appropriée, collé sur les compartiments.

On se réfère maintenant aux figures 5 et 6.

Dans cet autre mode de réalisation, les compartiments 1, 2 et 3 sont reliés entre eux au moyen d'un support 50.

Ce support 50 est fixé sur la face arrière des compartiments 1 à 3, correspondant à leur face convexe. Dans ce cas, les compartiments 1 à 3 ne sont pas nécessairement articulés entre eux au niveau de leurs bords adjacents.

Comme indiqué en regard des figures 3 et 4, chaque compartiment comporte de préférence, sur sa face intérieure concave 13, 23, 33 des moyens 41, 42, 43 de protection anti-brûlures.

L'article réfrigérant selon l'invention est mis en place sur la jambe, par exemple en plaçant tout d'abord le compartiment central 2 au contact de l'arrière de la jambe J du patient, puis en repliant l'article sur le reste de la jambe.

L'article réfrigérant est maintenu en position grâce aux moyens de fixation 60, 70.

Dans un autre mode de réalisation, le support 50 pourrait être disposé sur la face intérieure concave des compartiments.

Enfin, les signes de référence insérés après les caractéristiques techniques figurant dans les revendications ont pour seul but de faciliter la compréhension de ces dernières et ne sauraient en limiter la portée.

## Revendications

1. Article réfrigérant pour la jambe comprenant au moins deux compartiments indépendants (1, 2, 3) de forme allongée, réalisés en une matière souple et reliés entre eux par leur plus grande dimension, de façon à pouvoir conformer ledit article à la forme de la jambe (J), ledit article comprenant en outre des moyens de fixation (60, 70) pour le maintenir en position sur la jambe (J), caractérisé en ce que les compartiments sont étanches, chacun d'eux définissant une enceinte susceptible de recevoir directement un fluide et comportant un moyen de fermeture (11, 21, 31) amovible de ladite enceinte.

2. Article réfrigérant selon la revendication 1, caractérisé en ce que des cloisons (25) sont prévues dans au moins un compartiment à proximité d'un bord transversal (4) destiné à être situé à la partie inférieure de la jambe (J) lorsque l'article réfrigérant est en place.

3. Article réfrigérant selon la revendication 1 ou 2, dans lequel lesdits compartiments (1, 2, 3) sont fixés sur un support souple (50).

4. Article réfrigérant selon l'une des revendication 1 à 3, dans lequel lesdits compartiments sont articulés entre eux selon leur plus grande dimension.

5. Article selon l'une des revendications 1 à 4, dans lequel la face interne (13, 23, 33) des compartiments, destinée à être en contact avec la jambe, est recouverte d'une protection anti-brûlures (40 ; 41, 42, 43).

6. Article réfrigérant selon l'une des revendications 1 à 5, dans lequel lesdits moyens de fixation (60, 70) sont placés aux extrémités libres dudit support (50) ou de ladite protection anti-brûlures (40).

7. Article réfrigérant selon la revendication 6, dans lequel lesdits moyens de fixation sont constitués par deux bandes longitudinales du type velours crochet présentant une forme légèrement concave.

8. Article réfrigérant selon l'une quelconque des revendications précédentes, dans lequel lesdits compartiments présentent une forme légèrement courbée.

9. Procédé d'utilisation de l'article réfrigérant selon l'une quelconque des revendications 1 à 8, consistant, avant mise en place sur la jambe, à :
- remplir les compartiments (1, 2, 3) dudit article avec un fluide et fermer lesdits compartiments et
- placer ledit article dans un réfrigérateur ou congélateur pour refroidir ledit fluide.

10. Procédé d'utilisation selon la revendication 9, dans lequel le fluide utilisé pour remplir lesdits compartiments est un liquide tel que de l'eau, un mélange d'eau et d'alcool ou un liquide de refroidissement du type glycol.
